# EUROPEAN PATENT APPLICATION

(11) **EP 3 816 286 A1**
(43) Date of publication of application: **05.05.2021**
(21) Application number: 19819185.0
(22) Date of filing: 12.06.2019
(51) Int. Cl.: C12N 15/113, A61K 31/7088, A61K 48/00, A61P 9/00, A61P 43/00

(54) **MYOCARDIAL DYSFUNCTION THERAPEUTIC AGENT**

(30) Priority: 13.06.2018 JP 2018112863
(71) Applicant: Daiichi Sankyo Company, Limited, Tokyo 103-8426 (JP); Kobe Gakuin Educational Foundation, Kobe-shi, Hyogo 650-8586 (JP)
(72) Inventor: MATSUO, Masafumi, Kobe-shi, Hyogo 651-2180 (JP); KOIZUMI, Makoto, Tokyo 103-8426 (JP); ONISHI, Yoshiyuki, Tokyo 103-8426 (JP); SHOJI, Takao, Tokyo 103-8426 (JP)
(74) Representative: J A Kemp LLP
(86) International application number: PCT/JP2019/023265
(87) International publication number: WO 2019/240164

(57) **Abstract**

The present invention establishes a method for treating cardiac dysfunction. An oligonucleotide of 15-30 bp comprising a nucleotide sequence complementary to a part of the intron 55 region of a dystrophin gene, which comprises the sequence of 5'-TGTCTTCCT-3' or 5'-CAGCTTGAACCGGGC-3' (SEQ ID NO: 64) (wherein "T" may be "U" in either sequence), a pharmacologically acceptable salt thereof, or a solvate thereof. A prophylactic and/or a therapeutic for cardiac dysfunction, comprising the above-described oligonucleotide, a pharmacologically acceptable salt thereof, or a solvate thereof. A suppressor of Dp116 expression, comprising the above-described oligonucleotide, a pharmacologically acceptable salt thereof, or a solvate thereof.

## Description

### TECHNICAL FIELD

The present invention relates to a therapeutic for myocardial damage. More specifically, the present invention relates to an oligonucleotide capable of suppressing the expression of Dp116 mRNA, as well as a pharmaceutical drug containing the oligonucleotide.

### BACKGROUND ART

Duchenne muscular dystrophy (DMD) is caused by dystrophin deficiency because of mutations in the DMD gene. In most cases of DMD, cardiac dysfunction is involved and a large number of patients die from heart failure (Non-Patent Documents Nos. 1 and 2). As a therapy of this cardiac dysfunction in DMD, general cardioprotective agents and the like are used and no therapy of cardiac dysfunction is available that is specific to DMD (Non-Patent Document No. 3).

Exactly speaking, the dystrophin referred to above is a dystrophin isoform designated Dp427. Depending on the site of abnormality in the *DMD* gene, individual patients with DMD lack different isoforms of dystrophin. It is known that dystrophin has isoforms of Dp427, Dp260, Dp140, Dp116 and Dp71.

Matsuo et al. have found and reported that a causative factor for the onset of cardiac dysfunction in DMD resides in dystrophin Dp116 (hereinafter, sometimes referred to simply as "Dp116") that is expressed in the heart (Non-Patent Document No. 4). Analyses of relationships between Dp116 expression and cardiac dysfunction revealed that cardiac dysfunction occurred earlier and more severely in DMD patients with than without expression of Dp116. This result suggested that suppressing the expression of Dp116 is a molecular target in the treatment of cardiac dysfunction.

### PRIOR ART LITERATURE

### Non-Patent Documents

Non-Patent Document No. 1: Kamdar F, Garry DJ. Dystrophin-deficient cardiomyopathy. J Am Coll Cardiol. 2016; 67: 2533-2546.
Non-Patent Document No. 2: Nigro G, Comi LI, Politano L, Bain RJ. The incidence and evolutive of cardiomyopathy in Duchenne muscular dystrophy. Int J Cardiol. 1990; 26: 271-277.
Non-Patent Document No. 3: Markham LW, Spicer RL, Khoury PR, Wong BL, Mathews KD, Cripe LH. Steroid therapy and cardiac function in Duchenne muscular dystrophy. Pediatr Cardiol. 2005; 26:768-771.
Non-Patent Document No. 4: Yamamoto T, Awano H, Zhan Z, Enomoto-Sakuma M, Kitaaki S, Matsumoto M, Nagai M, Sato I, Imanishi T, Hayashi N, Matsuo M, Iijima K, Saegusa J, Cardiac dysfunction in Duchenne muscular dystrophy is less frequent in patients with mutations in the dystrophin Dp116 coding region than in other regions. 2018; Cirs Genom Precis Med. 2018; 11: e001782.

### DISCLOSURE OF THE INVENTION

### PROBLEM FOR SOLUTION BY THE INVENTION

It is an object of the present invention to establish a method for treating cardiac disorder.

### MEANS TO SOLVE THE PROBLEM

The present inventors have searched for modified nucleic acids capable of suppressing the expression of Dp116 mRNA. Briefly, various types of modified nucleic acid targeting to splicing enhancer sequence for Dp116 exon S1 were synthesized and introduced into U251 cells. Subsequently, the level of Dp116 mRNA was analyzed by RT-PCR. As a result, the present inventors have found a nucleic acid drug capable of inhibiting the expression of Dp116.

The nucleic acid therapeutics of the present invention is applicable to a therapy of cardiac dysfunction in DMD Further, it is believed that the nucleic acid therapeutics of the present invention is also applicable to a therapy of cardiac dysfunction in adults.

A summary of the present invention is as described below.
(1) An oligonucleotide, a pharmacologically acceptable salt thereof, or a solvate thereof, wherein the oligonucleotide having 15-30 base comprises a nucleotide sequence complementary to a part of the intron 55 region of a dystrophin gene, and comprises the sequence of 5'-TGTCTTCCT-3' or 5'-CAGCTTGAACCGGGC-3' (SEQ ID NO: 64) (wherein "T" may be "U" in either sequence).
(2) The oligonucleotide, a pharmacologically acceptable salt thereof of (1) above, which comprises the sequence of 5'-TGTCTTCCT-3' (wherein "T" may be "U").
(3) The oligonucleotide, a pharmacologically acceptable salt thereof, or a solvate thereof of (1) or (2) above, which comprises any one of the sequences of SEQ ID NOS: 15 to 59 (wherein "T" may be "U", and "U" may be "T").
(4) The oligonucleotide, a pharmacologically acceptable salt thereof, or a solvate thereof of (1) or (2) above, which comprises any one of the sequences of SEQ ID NOS: 20, 25 to 33 and 35 to 37.
(5) The oligonucleotide, a pharmacologically acceptable salt thereof, or a solvate thereof of any one of (1) to (4) above, which is capable of suppressing the expression of dystrophin Dp116.
(6) The oligonucleotide, a pharmacologically acceptable salt thereof, or a solvate thereof of any one of (1) to (5) above, wherein at least one of the sugar and/or the phosphodiester bond constituting the oligonucleotide is modified.
(7) The oligonucleotide, a pharmacologically acceptable salt thereof, or a solvate thereof of any one of (1) to (6) above, wherein the sugar constituting the oligonucleotide is D-ribofuranose and modification of the sugar is modification of the hydroxy group at 2'-position of D-ribofuranose.
(8) The oligonucleotide, a pharmacologically acceptable salt thereof, or a solvate thereof of any one of (1) to (7) above, wherein the sugar constituting the oligonucleotide is D-ribofuranose and modification of the sugar is 2'-O-alkylation and/or 2'-,4'-bridge of D-ribofuranose.
(9) The oligonucleotide, a pharmacologically acceptable salt thereof, or a solvate thereof of any one of (1) to (8) above, wherein the sugar constituting the oligonucleotide is D-ribofuranose and modification of the sugar is 2'-O-alkylation and/or 2'-O,4'-C-alkylenation of D-ribofuranose.
(10) The oligonucleotide, a pharmacologically acceptable salt thereof, or a solvate thereof of any one of (1) to (8) above, wherein the sugar constituting the oligonucleotide is D-ribofuranose and modification of the sugar is 2'-O-methylation and/or 2'-O,4'-C-ethylenation of D-ribofuranose.
(11) The oligonucleotide, a pharmacologically acceptable salt thereof, or a solvate thereof of any one of (1) to (10) above, wherein modification of the phosphodiester bond constituting the oligonucleotide is a phosphorothioate bond.
(12) A prophylactic and/or a therapeutic agent for cardiac dysfunction, comprising the oligonucleotide, a pharmacologically acceptable salt thereof, or a solvate thereof of any one of (1) to (11) above.
(13) The prophylactic and/or therapeutic agent of (12) above, which is to be applied to patients expressing dystrophin Dp116.
(14) The prophylactic and/or therapeutic agent of (13) above, wherein the patients expressing dystrophin Dp116 are patients with Duchenne muscular dystrophy.
(15) A suppressor of Dp116 expression, comprising the oligonucleotide, a pharmacologically acceptable salt thereof, or a solvate thereof of any one of (1) to (11) above.
(16) A method of preventing and/or treating cardiac dysfunction, comprising administering to a subject a pharmacologically effective amount of the oligonucleotide, a pharmacologically acceptable salt thereof, or a solvate thereof of any one of (1) to (11) above.
(17) A method of suppressing the expression of Dp116, comprising treating a Dp116 expressing cell, tissue or organ with the oligonucleotide, a pharmacologically acceptable salt thereof, or a solvate thereof of any one of (1) to (11) above.
(18) The oligonucleotide, a pharmacologically acceptable salt thereof, or a solvate thereof of any one of (1) to (11) above, for use in a method of preventing and/or treating cardiac dysfunction.
(19) Use of the oligonucleotide, a pharmacologically acceptable salt thereof, or a solvate thereof of any one of (1) to (11) above, for suppressing the expression of Dp116.
(20) A formulation for oral or parenteral administration, comprising the oligonucleotide, a pharmacologically acceptable salt thereof, or a solvate thereof of any one of (1) to (11) above.
(21) The oligonucleotide, a pharmacologically acceptable salt thereof, or a solvate thereof of any one of (1) to (11) above, for use as a pharmaceutical drug.

It is much expected that the present invention will provide a therapeutic method of extremely high specificity that targets Dp116 expressed by DMD patients.

### EFFECT OF THE INVENTION

According to the present invention, the expression of Dp116 can be suppressed to thereby treat cardiac dysfunction.

The present specification encompasses the contents disclosed in the specifications and/or drawings of Japanese Patent Application No. 2018-112863 based on which the present patent application claims priority.

### BRIEF DESCRIPTION OF THE DRAWINGS

[Fig. 1] Synthesized antisense oligonucleotides were introduced into U251 cells. After 24 hours, mRNA extracted from the cells was amplified by RT-PCR and analyzed with Bioanalyzer. Electrophoretic bands from RT-PCR products of Dp116 and GAPDH are shown.
[Fig. 2] Results of semi-quantitative analysis of the products amplified in Fig. 1. RT-PCR product ratios of Dp116 to GAPDH were determined. When AO was not added (MQ), the ratio was regarded as 1. Values for the respective AO additions are shown in ratios.
[Fig. 3] The mode of action of the oligonucleotide of the present invention is illustrated. The oligonucleotide of the present invention blocks the splicing reaction that ligates exon 1 (S1) and exon 56 in Dp116, whereby the expression of Dp116 can be specifically suppressed.
[Fig. 4] This figure shows the suppression of Dp116 protein expression in MiraCell cardiomyocytes by compounds of Examples. Vertical axis represents the band strength of Dp116 divided by the band strength of β-actin.
[Fig. 5] This figure shows the suppression of Dp116 mRNA expression in U251 cells by compounds of Examples. Vertical axis represents the band strength of Dp116's PCR product divided by the band strength of GAPDH's PCR product.
[Fig. 6] This figure shows the suppression of Dp116 mRNA expression in U251 cells by compounds of Examples. Vertical axis represents the band strength of Dp116's PCR product divided by the band strength of GAPDH's PCR product.

### BEST MODES FOR CARRYING OUT THE INVENTION

Hereinbelow, the embodiment of the present invention will be described in detail.

The present invention provides an oligonucleotide of 15-30 base comprising a nucleotide sequence complementary to a part of the intron 55 region of a dystrophin gene, wherein the oligonucleotide comprises the sequence of 5'-TGTCTTCCT-3' or 5'-CAGCTTGAACCGGGC-3' (SEQ ID NO: 64) (wherein "T" may be "U" in either sequence), a pharmacologically acceptable salt thereof, or a solvate thereof.

Preferably, the oligonucleotide of the present invention, pharmacologically acceptable salts thereof and solvates thereof comprise the sequence of 5'-TGTCTTCCT-3' (wherein "T" may be "U").

The oligonucleotide of the present invention, pharmacologically acceptable salts thereof and solvates thereof may be exemplified by those which comprise any one of the sequences of SEQ ID NOS: 15 to 59 (wherein "T" may be "U", and "U" may be "T").

The number of bases in the oligonucleotide of the present invention is suitably 15-30, preferably 15-21, and more preferably 15-18.

The oligonucleotide of the present invention, pharmacologically acceptable salts thereof and solvates thereof may be suitably those which are capable of suppressing the expression of dystrophin Dp116. Prophylactic and/or therapeutic effect on cardiac dysfunction can be expected by suppressing the expression of Dp116.

The *DMD* gene is the responsible gene for Duchenne muscular dystrophy (DMD). Due to abnormality in the *DMD* gene, dystrophin (Dp427) deficiency is caused. *DMD* is the largest human gene 4200 kb in size that encodes a 14 kb mRNA consisting of 79 exons. Five promoters are located within this gene, producing tissue-specific dystrophin isoforms of Dp427, Dp260, Dp140, Dp116 and Dp71, respectively.

Dystrophin Dp116 is the second smallest isoform produced from the promoter located in intron 55 and is expressed in Schwann cells. This promoter is designated as "S promoter", and exon 1 as "SI". The mRNA of Dp116 shares Dp116 specific exon 1 and *DMD*'s exons 56 to 79 with other isoforms.

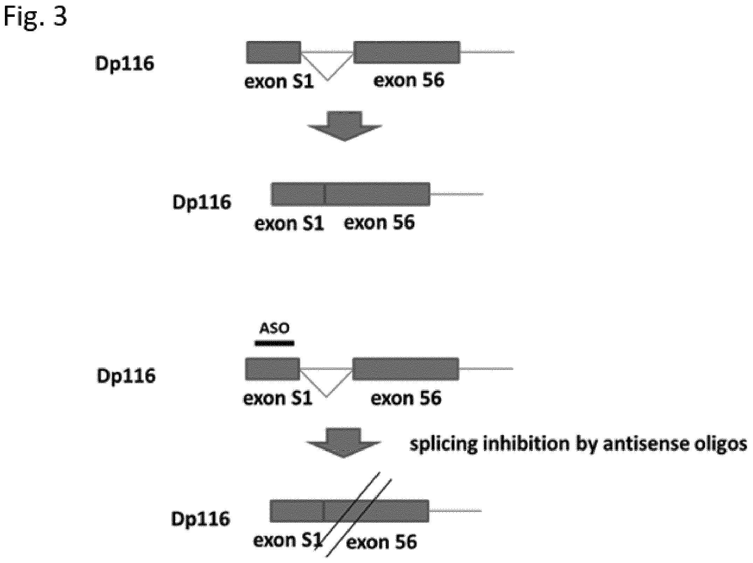

The oligonucleotide of the present invention is an antisense oligonucleotide comprising a nucleotide sequence complementary to a part of the intron 55 region of a dystrophin gene. The oligonucleotide of the present invention is capable of blocking the splicing reaction that ligates exon 1 (S1) and exon 56 in Dp116, whereby the expression of Dp116 can be specifically suppressed (Fig. 3). The oligonucleotide of the present invention is believed to cause no or little effect, if any, on the function of intron 55; for example, it is believed that the oligonucleotide of the present invention will not degrade the pre-mRNA of Dp427.

Nucleotides constituting the oligonucleotide (antisense oligonucleotide) of the present invention may be either natural DNA, natural RNA, chimera DNA/RNA, or modified DNA, RNA or DNA/RNA. Preferably, at least one of the nucleotides is a modified nucleotide.

Examples of modified nucleotides of the present invention include those in which a sugar is modified (e.g., the hydroxy group at 2'-position of D-ribofuranose is modified (D-ribofuranose is 2'-O-alkylated or D-ribofuranose is 2'-,4'-bridged (e.g., D-ribofuranose is 2'-O,4'-C-alkylenated), etc.), those in which a phosphodiester bond is modified (e.g., thioated), those in which a base is modified, combinations of the above-described nucleotides, and so forth. Antisense oligonucleotides in which at least one D-ribofuranose constituting the oligonucleotides is 2'-O-alkylated or 2'-O,4'-C-alkylenated have high RNA binding strength and high resistance to nuclease. Thus, they are expected to produce higher therapeutic effect than natural nucleotides (i.e. oligo DNA or oligo RNA). Further, oligonucleotides in which at least one phosphodiester bond constituting the oligonucleotides is thioated also have high resistance to nuclease and, thus, are expected to produce higher therapeutic effect than natural nucleotides (i.e. oligo DNA or oligo RNA). Oligonucleotides comprising both the modified sugar and the modified phosphate as described above have even higher resistance to nuclease and, thus, are expected to produce even higher therapeutic effect.

With respect to the oligonucleotide (antisense oligonucleotide) of the present invention, examples of modified sugars include, but are not limited to, D-ribofuranose as 2'-O-alkylated (e.g. 2'-O-methylated, 2'-O-aminoethylated, 2'-O-propylated, 2'-O-allylated, 2'-O-methoxyethylated, 2'-O-butylated, 2'-O-pentylated, or 2'-O-propargylated); D-ribofuranose as 2'-O,4'-C-alkylenated (e.g. 2'-O,4'-C-ethylenated, 2'-O,4'-C-methylenated, 2'-O,4'-C-propylenated, 2'-O,4'-C-tetramethylenated, or 2'-O,4'-C-pentamethylenated); D-ribofuranose as 2'-,4'-bridged, for example, S-cEt (2',4'-constrained ethyl), AmNA (Amide-bridged nucleic acid), etc.; D-ribofuranose as 2'-deoxy-2'-C,4'-C-methyleneoxymethylated, or D-ribofuranose as 2' deoxygenated in combination with other modifications, for example, 3'-deoxy-3'-amino-2'-deoxy-D-ribofuranose, 3'-deoxy-3'-amino-2'-deoxy-2'-fluoro-D-ribofuranose, etc.

With respect to the oligonucleotide (antisense oligonucleotide) of the present invention, examples of the modification of phosphodiester bond include, but are not limited to, phosphorothioate bond, methylphosphonate bond, methylthiophosphonate bond, phosphorodithioate bond and phosphoroamidate bond.

With respect to the oligonucleotide (antisense oligonucleotide) of the present invention, examples of modified bases include, but are not limited to, cytosine as 5-methylated, 5-fluorinated, 5-brominated, 5-iodinated or N4-methylated; thymine as 5-demethylated (uracil), 5-fluorinated, 5-brominated or 5-iodinated; adenine as N6-methylated or 8-brominated; and guanine as N2-methylated or 8-brominated.

Nucleotide residues constituting the oligonucleotide of the present invention include A^{t}, G^{t}, 5meC^{t}, C^{t}, T^{t}, U^{t}, A^{p}, G^{p}, 5meC^{p}, C^{p}, T^{p}, U^{p}, A^{s}, G^{s}, 5meC^{s}, C^{s}, T^{s}, U^{s}, A^{mlt}, G^{mlt} , C^{mlt}, 5meC^{mlt}, U^{mlt}, A^{mlp}, G^{mlp}, C^{mlp}, 5meC^{mlp}, U^{mlp}, A^{mls}, G^{mls}, C^{mls}, 5meC^{mls}, U^{mls}, A^{2t}, G^{2t}, C^{2t}, T^{2t}, A^{e2p}, G^{e2p}, C^{e2p}, T^{e2p}, A^{e2s}, G^{e2s}, C^{e2s}, T^{e2s}, A^{lt}, G^{lt}, C^{lt}, T^{lt}, A^{elp}, G^{elp}, C^{elp}, T^{elp}, A^{els}, G^{els}, C^{els}, T^{els}, A^{m2t}, G^{m2t}, 5meC^{m2t}, T^{m2t}, A^{m2p}, G^{m2p}, 5meC^{m2p}, T^{m2p}, A^{m2s}, G^{m2s}, 5meC^{m2s} and T^{m2s}, the structures of which are shown below:

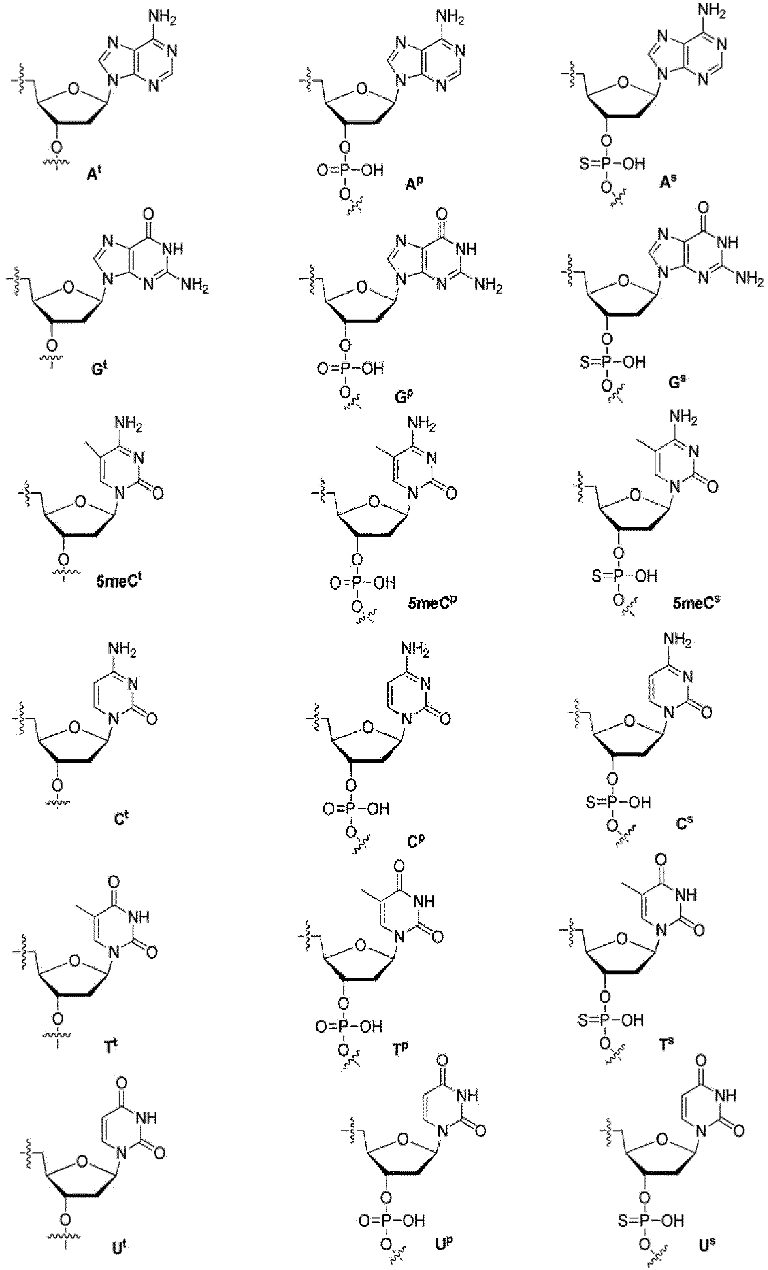

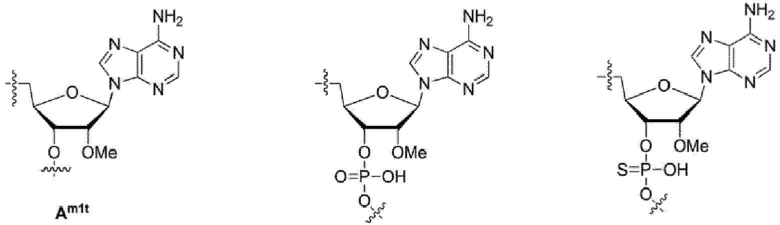

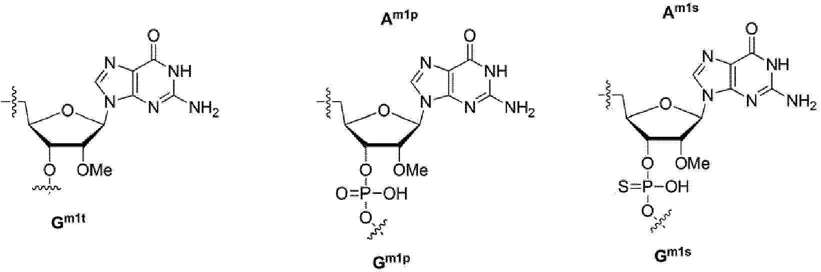

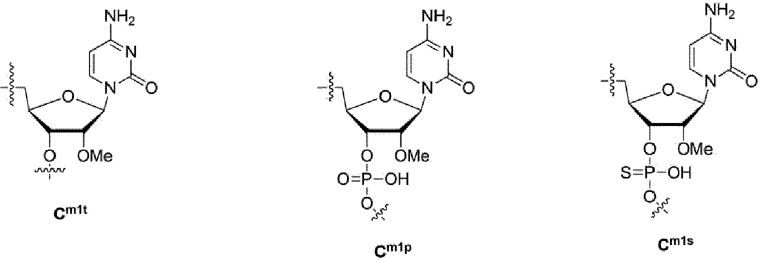

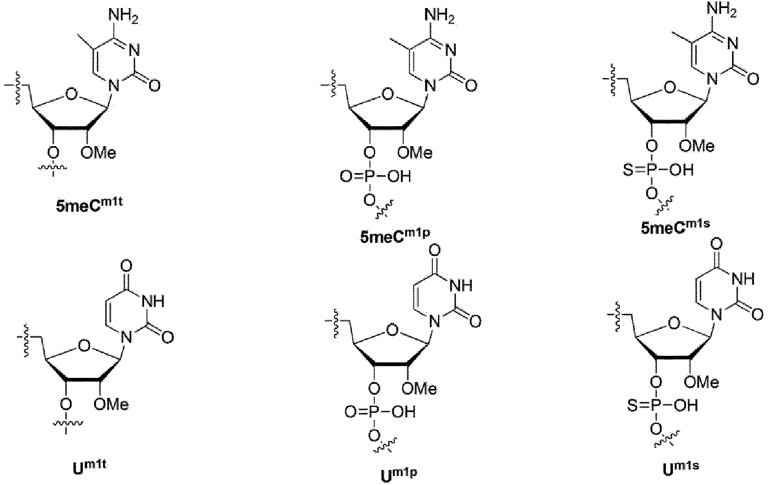

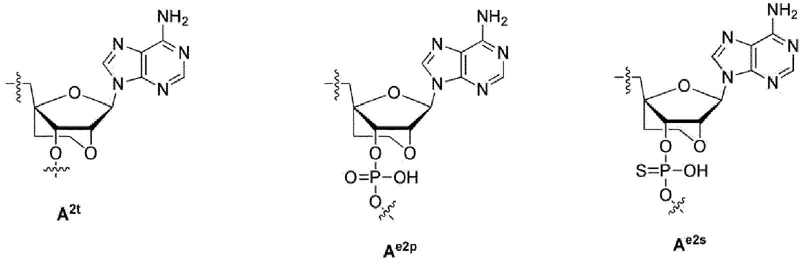

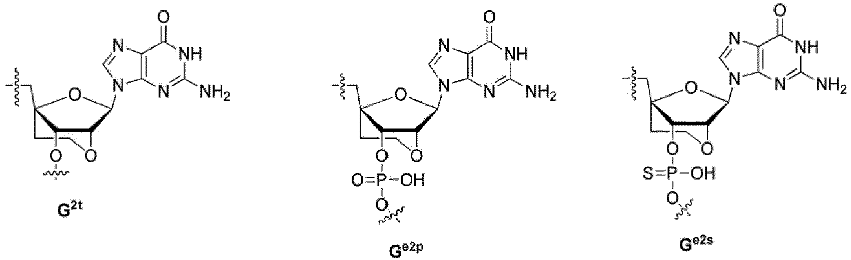

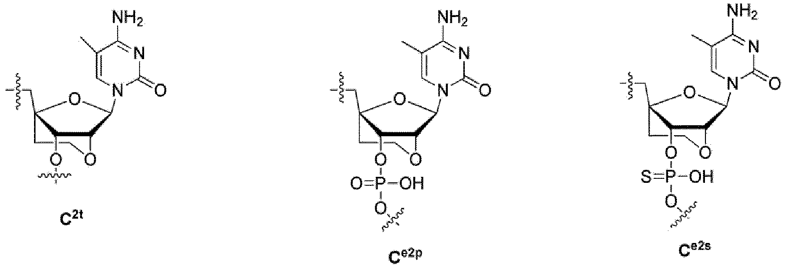

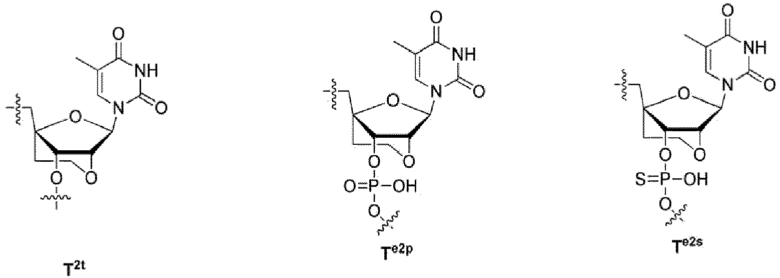

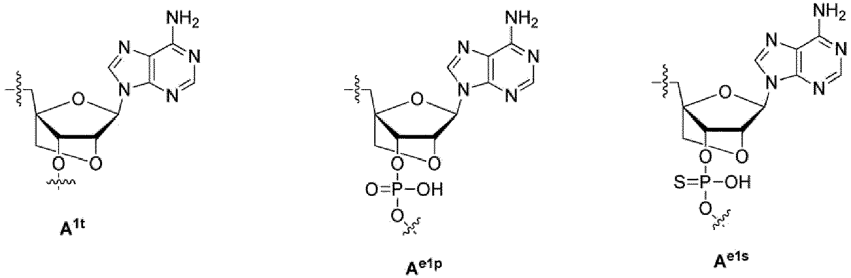

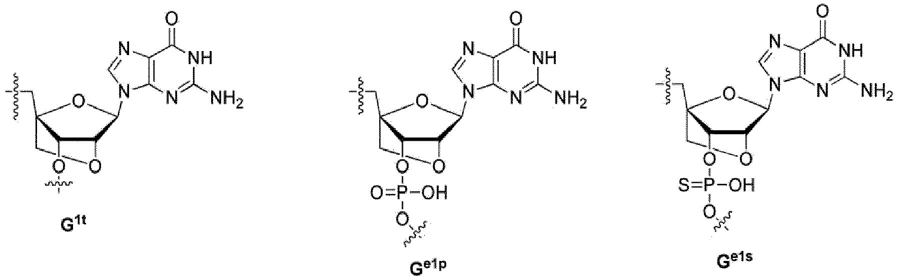

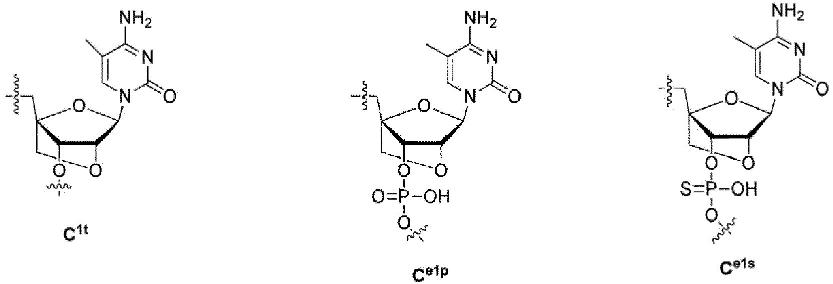

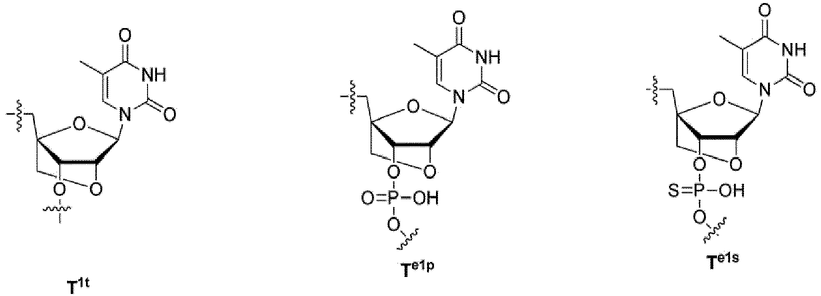

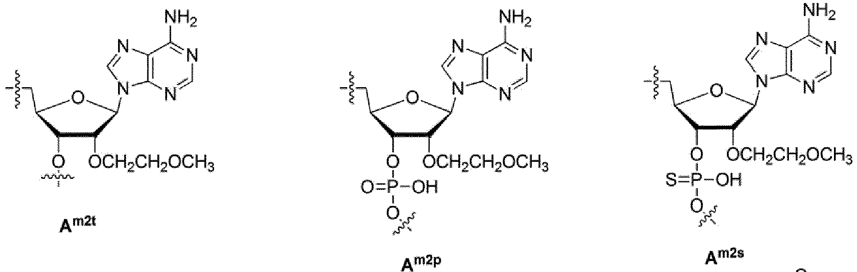

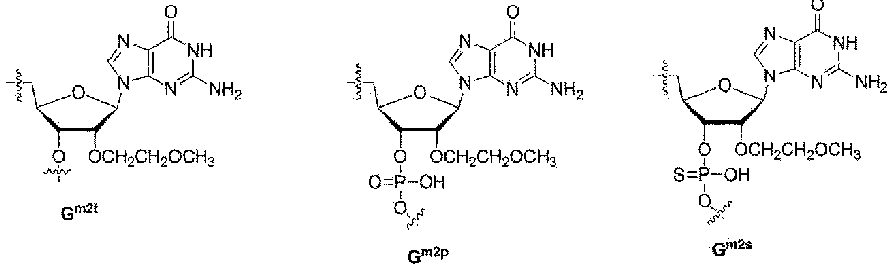

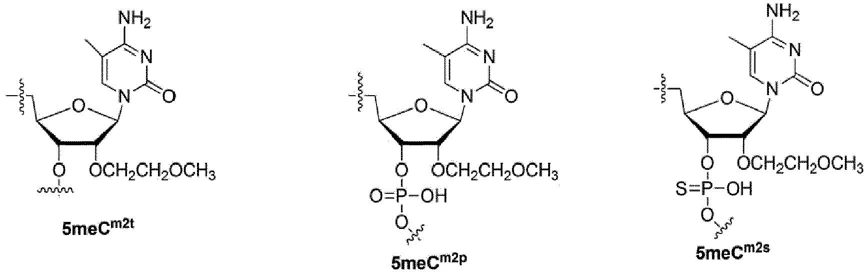

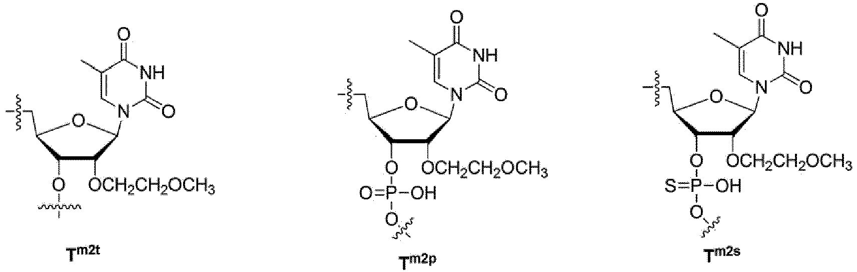

The oligonucleotide (antisense oligonucleotide) of the present invention may be synthesized with a commercially available DNA synthesizer (e.g., PerkinElmer Model 392 based on the phosphoramidite method) according to the method described in Nucleic Acids Research, 12, 4539 (1984) with necessary modifications. As phosphoramidite reagents to be used in the process, natural nucleosides and 2'-O-methylnucleosides (i.e., 2'-O-methylguanosine, 2'-O-methyladenosine, 2'-O-methylcytidine and 2'-O-methyluridine) are commercially available. As regards 2'-O-alkylguanosine, -alkyladenosine, -alkylcytidine and -alkyluridine in which the carbon number of the alkyl group is 2-6, the following methods may be employed.

2'-O-aminoethylguanosine, -aminoethyladenosine, -aminoethylcytidine and -aminoethyluridine may be synthesized as previously described (Blommers et al., Biochemistry (1998),37, 17714-17725).

2'-O-propylguanosine, -propyladenosine, -propylcytidine and -propyluridine may be synthesized as previously described (Lesnik, E.A. et al., Biochemistry (1993), 32, 7832-7838).

For the synthesis of 2'-O-allylguanosine, -allyladenosine, -allylcytidine and -allyluridine, commercially available reagents may be used.

2'-O-methoxyethylguanosine, -methoxyethyladenosine, -methoxyethylcytidine and -methoxyethyluridine may be synthesized as previously described (US Patent No. 6261840 or Martin, P. Helv. Chim. Acta. (1995) 78, 486-504).

2'-O-butylguanosine, -butyladenosine, -butylcytidine and -butyluridine may be synthesized as previously described (Lesnik, E.A. et al., Biochemistry (1993), 32, 7832-7838).

2'-O-pentylguanosine, -pentyladenosine, -pentylcytidine and -pentyluridine may be synthesized as previously described (Lesnik, E.A. et al., Biochemistry (1993), 32, 7832-7838).

For the synthesis of 2'-O-propargylguanosine, -propargyladenosine, -propargylcytidine and -propargyluridine, commercially available reagents may be used.

2'-O,4'-C-methyleneguanosine, 2'-O,4'-C-methyleneadenosine, 2'-O,4'-C-methylenecytidine, 5-methylcytidine and 5-methylthymidine may be prepared according to the method described in WO99/14226; and 2'-O,4'-C-alkyleneguanosine, 2'-O,4'-C-alkyleneadenosine, 2'-O,4'-C-methylenecytidine, 5-methylcytidine and 5-methylthymidine in which the carbon number of the alkylene group is 2-5 may be prepared according to the method described in WO00/47599.

Nucleosides in which D-ribofuranose is 2'-deoxy-2'-C,4'-C-methyleneoxymethylenated may be synthesized as previously described (Wang, G. et al., Tetrahedron (1999), 55, 7707-7724).

S-cEt (constrained ethyl) may be synthesized as previously described (Seth, P.P. et al. J. Org. Chem (2010), 75, 1569-1581).

AmNA may be synthesized as previously described (Yahara, A. et al. ChemBioChem (2012), 13, 2513-2516; or WO2014/109384).

In the present invention, nucleobase sequences may be described using the abbreviation (A) or (a) for adenine, (G) or (g) for guanine, (C) or (c) for cytosine, (T) or (t) for thymine, and (U) or (u) for uracil. Instead of cytosine, 5-methylcytosine may be used. Among the nucleobases, uracil (U) or (u) and thymine (T) or (t) are interchangeable. Both uracil (U) or (u) and thymine (T) or (t) may be used in base pairing with adenine (A) or (a) in the complementary strand.

An antisense oligonucleotide with phophorothioate bonds can be synthesized by coupling phosphoramidite reagents and then reacting sulfur, tetraethylthiuram disulfide (TETD; Applied Biosystems), Beaucage reagent (Glen Research) or a reagent such as xanthan hydride (Tetrahedron Letters, 32, 3005 (1991); J. Am. Chem. Soc. 112, 1253 (1990); PCT/WO98/54198).

As controlled pore glass (CPG) to be used in a DNA synthesizer, 2'-O-methylnucleoside-bound CPG is commercially available. As regards 2'-O,4'-C-methyleneguanosine, 2'-O,4'-C-methyleneadenosine, 5-methylcytidine and thymidine, they may be prepared according to the method described in WO99/14226; and as regards 2'-O,4'-C-alkyleneguanosine, adenosine, 5-methylcytidine and thymidine in which the carbon number of the alkylene group is 2-5, they may be prepared according to the method described in WO00/47599. The thus prepared nucleosides may then be bound to CPG as previously described (Oligonucleotide Synthesis, Edited by M. J. Gait, Oxford University Press, 1984). By using the modified CPG (as disclosed in Example 12b of Japanese Unexamined Patent Publication No. Hei7-87982), an oligonucleotide in which a 2-hydroxyethylphosphate group is bound at the 3' end can be synthesized. If 3'-amino-Modifier C3 CPG, 3'-amino-Modifier C7 CPG or Glyceryl CPG (Glen Research) or 3'-specer C3 SynBase CPG 1000 or 3'-specer C9 SynBase CPG 1000 (Link Technologies) is used, an oligonucleotide in which a hydroxyalkylphosphate group or aminoalkylphosphate group is bound at the 3' end can be synthesized.

The oligonucleotide (antisense oligonucleotide) of the present invention may be used as a pharmaceutical drug for preventing and/or treating cardiac dysfunction, in particular, myocardial damage (for example, cardiomyopathy, preferably dilated cardiomyopathy).

The oligonucleotide (antisense oligonucleotide) of the present invention may be used in the form of a pharmacologically acceptable salt. The term "pharmacologically acceptable salt" as used herein refers to salts of the oligonucleotide (antisense oligonucleotide). Examples of such salts include, but are not limited to, alkaline metal salts such as sodium salts, potassium salts or lithium salts; alkaline earth metal salts such as calcium salts or magnesium salts; metal salts such as aluminum salts, iron salts, zinc salts, copper salts, nickel salts or cobalt salts; amine salts including inorganic salts such as ammonium salts and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts or tris(hydroxymethyl)aminomethane salts; inorganic acid salts including hydrohalogenic acid salts such as hydrofluorides, hydrochlorides, hydrobromides or hydroiodides, as well as nitrates, perchlorates, sulfates or phosphates; organic acid salts including lower alkane sulfonic acid salts such as methanesulfonates, trifluoromethanesulfonates or ethanesulfonates, arylsulfonic acid salts such as benzenesulfonates or p-toluenesulfonates, as well as acetates, malates, fumarates, succinates, citrates, tartrates, oxalates or maleates; and amino acid salts such as glycine salts, lysine salts, arginine salts, ornithine salts, glutamic acid salts or aspartic acid salts. These salts may be prepared by known methods.

The oligonucleotide (antisense oligonucleotide) or a pharmacologically acceptable salt thereof sometimes occur as a solvate (e.g., hydrate). The oligonucleotide (antisense oligonucleotide) or a pharmacologically acceptable salt thereof of the present invention may be such a solvate.

Therefore, the present invention provides a prophylactic and/or a therapeutic for cardiac dysfunction, comprising the above-described oligonucleotide, a pharmacologically acceptable salt thereof, or a solvate thereof.

The prophylactic and/or therapeutic of the present invention may be applied to patients expressing dystrophin Dp116. Suitably, patients expressing dystrophin Dp116 may be patients with DMD, to whom the present invention is by no means limited.

When the oligonucleotide (antisense oligonucleotide) of the present invention, a pharmacologically acceptable salt thereof or a solvate thereof is used for prevention and/or treatment of cardiac dysfunction, they may be administered *per se* or mixed with appropriate, pharmacologically acceptable excipients, diluents, and the like for oral administration in the form of tablets, capsules, granules, powders, syrups, etc. or for parenteral administration in the form of injections, suppositories, patches or external preparations.

These formulations may be prepared by well-known methods using additives such as excipients (e.g., organic excipients including sugar derivatives such as lactose, sucrose, glucose, mannitol or sorbitol; starch derivatives such as corn starch, potato starch, α-starch or dextrin; cellulose derivatives such as crystalline cellulose; gum arabic; dextran; or pullulan; and inorganic excipients including silicate derivatives such as light anhydrous silicic acid, synthetic aluminum silicate, calcium silicate or magnesium aluminometasilicate; phosphates such as calcium hydrogenphosphate; carbonates such as calcium carbonate; and sulfates such as calcium sulfate), lubricants (e.g., stearic acid; metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate or magnesium lauryl sulfate; silicic acid compounds such as silicic anhydride and silicic hydrate; or the starch derivatives listed above), binders (e.g., hydroxypropylcellulose, hydroxypropylmethylcellulose, polyvinylpyrrolidone, macrogol, or compounds similar to the above-listed excipients), disintegrants (e.g., cellulose derivatives such as low-substituted hydroxypropylcellulose, carboxymethylcellulose, calcium carboxymethylcellulose or internally crosslinked sodium carboxymethylcellulose; and chemically modified starch/cellulose derivatives such as carboxymethylstarch, sodium carboxymethylstarch or crosslinked polyvinylpyrrolidone), emulsifiers (e.g., colloidal clay such as bentonite or veegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylenealkylether, polyoxyethylene sorbitan fatty acid ester or sucrose esters of fatty acids), stabilizers (e.g., p-hydroxybenzoate esters such as methylparaben or propylparaben; alcohols such as chlorobutanol, benzyl alcohol or phenyl ethyl alcohol; benzalkonium chloride; phenols such as phenol or cresol; thimerosal; dehydroacetic acid; or sorbic acid), flavoring agents (e.g., conventionally used sweeteners, acidifiers, flavors and the like) or diluents.

The prophylactic and/or therapeutic of the present invention may comprise 0.1-250 µmoles/ml, preferably 1-50 µmoles/ml of oligonucleotide (antisense oligonucleotide), pharmacologically acceptable salt thereof or solvate thereof; 0.02-10% w/v of carbohydrate or polyalcohol; and 0.01-0.4% w/v of pharmacologically acceptable surfactant.

As the above carbohydrate, monosaccharides or disaccharides are especially preferable. Specific examples of these carbohydrates and polyalcohols include, but are not limited to, glucose, galactose, mannose, lactose, maltose, mannitol and sorbitol. These may be used alone or in combination.

Preferable examples of the surfactant include, but are not limited to, polyoxyethylene sorbitan mono-, di- or tri-ester, alkylphenylpolyoxyethylene, sodium taurocholate, sodium cholate and polyalcohol esters. Among these, polyoxyethylene sorbitan mono-, di- and tri-ester are especially preferable; the most preferable esters are oleate, laurate, stearate and palmitate. These may be used alone or in combination.

More preferably, the prophylactic and/or therapeutic drug of the present invention may comprise 0.03-0.09 M pharmacologically acceptable neutral salt such as sodium chloride, potassium chloride and/or calcium chloride.

Even more preferably, the prophylactic and/or therapeutic drug of the present invention may comprise 0.002-0.05 M pharmacologically acceptable buffer. Examples of a preferable buffer include, but are not limited to, sodium citrate, sodium glycinate, sodium phosphate and tris(hydroxymethyl)aminomethane. These buffers may be used alone or in combination.

Further, the above-described drug may be supplied in a state of solution. However, as in the case where there is a need for storage over a certain period of time, the drug is preferably lyophilized for stabilizing the oligonucleotide (antisense oligonucleotide) to thereby prevent the lowering of its therapeutic effect. When lyophilized, the drug may be reconstructed with a solution, such as distilled water for injection, just before use. Thus, the drug is returned into the state of a liquid to be administered. Therefore, the prophylactic and/or therapeutic drug of the present invention encompasses one in a lyophilized state that is used after reconstruction with a solution so that the respective components fall within specified concentration ranges. For the purpose of promoting the solubility of the lyophilized product, the drug may further comprise albumin and amino acids such as glycine.

When the oligonucleotide (antisense oligonucleotide) of the invention, a pharmacologically acceptable salt thereof or a solvate thereof is administered to a human, the oligonucleotide or the like may be administered, for example, at approximately 0.01-100 mg/kg (body weight), preferably at 0.1-20 mg/kg (body weight) per adult per day either once or over several times by subcutaneous injection, intravenous infusion or intravenous injection. The dose and the number of times of administration may be changed appropriately depending on the type and symptoms of the disease, the age of the patient, administration route, etc.

Administration of the oligonucleotide (antisense oligonucleotide) of the invention, a pharmacologically acceptable salt thereof or a solvate thereof to patients expressing dystrophin Dp116 may be performed, for example, as described below. Briefly, the antisense oligonucleotide, pharmacologically acceptable salt thereof or solvate thereof is prepared by methods well-known to one of ordinary skill in the art and sterilized by conventional methods to prepare, for example, 125 mg/ml of an injection solution. This solution is instilled to a patient intravenously in the form of, for example, infusion so that the dose of the oligonucleotide (antisense oligonucleotide) is, for example, 10 mg per kg body weight. This administration is repeated, for example, at 1-week intervals. Subsequently, this treatment is appropriately repeated while confirming the therapeutic effect by echocardiography or the like.

Further, the oligonucleotide (antisense oligonucleotide) of the invention, a pharmacologically acceptable salt thereof or a solvate thereof may be used for suppressing the expression of Dp116. Therefore, the present invention provides a suppressor of Dp116 expression, comprising the oligonucleotide of the invention, a pharmacologically acceptable salt thereof, or a solvate thereof. As the suppressor of Dp116 expression, an oligonucleotide of the invention capable of reducing the Dp116 mRNA level in Dp116 expressing cells to approximately 30% or less, preferably 20% or less, and more preferably 10% or less, relative to the level in control cells, a pharmacologically acceptable salt thereof, or a solvate thereof may be used. However, as long as an oligonucleotide of the invention has the ability to suppress Dp116 expression, the oligonucleotide, a pharmacologically acceptable salt thereof, or a solvate thereof may be used even if they do not satisfy the above-specified ranges. The suppressor of Dp116 expression according to the present invention may be used as a pharmaceutical drug or a reagent for experiments.

When the suppressor of Dp116 expression according to the present invention is used as a reagent for experiments, the expression of Dp116 can be suppressed by treating Dp116 expressing cells, tissues or organs with the oligonucleotide (antisense oligonucleotide) of the present invention, a pharmacologically acceptable salt thereof, or a solvate thereof. The oligonucleotide (antisense oligonucleotide) of the present invention, a pharmacologically acceptable salt thereof, or a solvate thereof may be used in an amount effective for suppressing the expression of Dp116. The Dp116 expressing cells may be exemplified by naturally occurring cells such as glioblastoma-derived cells, cardiomyocytes, and Schwann cells. In addition to the naturally occurring cells, *Dp116* gene-transfected recombinant cells may also be employed. The Dp116 expressing tissues and organs may be exemplified by glioblastoma, heart, peripheral nervous system, etc. The expression of Dp116 may be analyzed by analyzing Dp116 mRNA in samples through RT-PCR, by detecting a Dp116 protein in samples through Western blotting, or by detecting Dp116 specific peptide fragments through mass spectrometry.

### EXAMPLES

Hereinbelow, the present invention will be described more specifically with reference to the following Examples. These Examples are given only for explanation purposes and are not intended to limit the scope of the present invention.

### [Reference Examples 1 to 14]

HO-A^{mls}-T^{e2s}-A^{mls}-G^{mls}-T^{e2s}-A^{mls}-G^{mls}-A^{e2s}-A^{mls}-G^{mls}-A^{e2s}-A^{mls}-U^{mls}-C^{e2s}-U^{mls}-U^{mls}-A^{e2s}-C^{mlt}-H (Dp116-01) (SEQ ID NO: 1)

Synthesis was performed with an automated nucleic acid synthesizer (BioAutomation's MerMade 192X) by the phosphoramidite method (Nucleic Acids Research, 12, 4539 (1984)). As reagents, Activator Solution-3 (0.25 mol/L 5-Benzylthio-1H-tetrazole-Acetonitrile Solution; Wako Pure Chemical; product No. 013-20011), Cap A for AKTA (1-Methylimidazole-Acetonitrile Solution; Sigma-Aldrich; product No. L040050), Cap B1 for AKTA (Acetic Anhydride-Acetonitrile Solution; Sigma-Aldrich; product No. L050050), Cap B2 for AKTA (Pyridine-Acetonitrile Solution; Sigma-Aldrich; product No. L050150), and DCA Deblock (Dichloroacetic Acid-Toluene Solution; Sigma-Aldrich; product No. L023050) were used. As a thiolation reagent for formation of phosphorothioate bond, phenylacetyl disulfide (Carbosynth; product No. FP07495) was dissolved in a 1:1 (v/v) solution of acetonitrile (dehydrated; Kanto Chemical Co., Inc.; product No. 01837-05) and pyridine (dehydrated; Kanto Chemical Co., Inc.; product No. 11339-05) to give a concentration of 0.2 M. As amidite reagents, 2'-O-Me nucleoside phosphoramidites (for adenosine: product No. ANP-5751; for cytidine: product No. ANP-5752; for guanosine: product No. ANP-5753; for uridine: product No. ANP-5754) were products from ChemGenes. Non-natural phosphoramidites used were the following compounds disclosed in the indicated Examples of Japanese Unexamined Patent Publication No. 2000-297097: Example 14 (5'-O-dimethoxytrityl-2'-O,4'-C-ethylene-6-N-benzoyladenosine-3'-O-(2-cyanoethyl N,N-diisopropyl)phosphoramidite); Example 27 (5'-O-dimethoxytrityl-2'-O,4'-C-ethylene-2-N-isobutylguanosine-3'-O-(2-cyanoethyl N,N-diisopropyl)phosphoramidite); Example 22 (5'-O-dimethoxytrityl-2'-O,4'-C-ethylene-4-N-benzoyl-5-methylcytidine-3'-O-(2-cyanoethyl N,N-diisopropyl)phosphoramidite); and Example 9 (5'-O-dimethoxytrityl-2'-O,4'-C-ethylene-5-methyluridine-3'-O-(2-cyanoethyl N,N-diisopropyl)phosphoramidite). As a solid-phase carrier, Glen Unysupport™ FC 96 well format 0.2 µmol (GlenResearch) was used. Thus, the compound of Reference Example 1 was synthesized. It should be noted here that about 9 minutes was set as the time required for condensation of amidites.

Protected oligonucleotide analogs with the sequence of interest were treated with 600 µl of thick aqueous ammonia to thereby cut out oligomers from the support and, at the same time, remove the protective group cyanoethyl on phosphorus atoms and the protective group on nucleobases. The resultant oligomer mixture in solution was mixed with 300 µl of Clarity QSP DNA Loading Buffer (Phenomenex) and charged on Clarity SPE 96 well plates (Phenomenex). One milliliter of Clarity QSP DNA Loading Buffer : water = 1:1 solution, 3 mL of water, 3 ml of 3% dichloroacetic acid (DCA) aqueous solution and 6 ml of water were added in this order. Subsequently, components extracted with a 9:1 solution of 20 mM Tris aqueous solution and acetonitrile were collected. After distilling off the solvent, the compound of interest was obtained. When analyzed by reversed-phase HPLC [column (Phenomenex, Clarity 2.6 µm Oligo-MS 100A(2.1x50 mm)), Solution A: an aqueous solution of 100 mM hexafluoroisopropanol (HFIP) and 8 mM trimethylamine, Solution B: methanol, B%: from 10% to 25% (4 min, linear gradient); 60°C; 0.5 mL/min; 260 nm)], the subject compound was eluted at 2.887 min. The compound was identified by negative-ion ESI mass spectrometry.

The nucleotide sequence of the subject compound is a sequence complementary to nucleotide Nos. 1836403 to 1836420 of Homo sapiens dystrophin (DMD) (NCBI-GenBank accession No. NG_012232.1).

The compounds of Reference Examples 2 to 14 were also synthesized in the same manner as for the compound of Reference Example 1. Data from Reference Examples 1 to 14 are summarized in Table 1 below.

**Table 1**

| Reference Example | Designation | Sequence (5'-3') | Start | End | Molecular Weight | SEQ ID NO: |
|---|---|---|---|---|---|---|
| 1 | Dp 116-01 | aTagTagAagAauCugAc | 1836403 | 1836420 | 6422.78 | 1 |
| 2 | Dp 116-02 | gTagAagAauCugAccTu | 1836400 | 1836417 | 6375.72 | 2 |
| 3 | Dp 116-03 | gAagAauCugAccTuuAc | 1836397 | 1836414 | 6321.64 | 3 |
| 4 | Dp 116-04 | gAauCugAccTuuAcaTg | 1836394 | 1836411 | 6312.64 | 4 |
| 5 | Dp116-05 | uCugAccTuuAcaTggTa | 1836391 | 1836408 | 6303.32 | 5 |
| 6 | Dp 116-06 | gAccTuuAcaTggTauGu | 1836388 | 1836405 | 6329.65 | 6 |
| 7 | Dp 116-07 | cTuuAcaTggTauGucTu | 1836385 | 1836402 | 6281.62 | 7 |
| 8 | Dp 116-07.1 | uTuaCauGguAugTcuTc | 1836384 | 1836401 | 6281.64 | 8 |
| 9 | Dp 116-07.2 | uTacAugGuaTguCuuCc | 1836383 | 1836400 | 6280.65 | 9 |
| 10 | Dp116-16 | cTucCugTguAacAuuTu | 1836370 | 1836387 | 6241.62 | 10 |
| 11 | Dp116-17 | cCugTguAacAuuTucAg | 1836367 | 1836384 | 6289.67 | 11 |
| 12 | Dp116-18 | gTguAacAuuTucAgcTu | 1836364 | 1836381 | 6290.65 | 12 |
| 13 | Dp116-19 | uAacAuuTucAgcTugAa | 1836361 | 1836378 | 6283.67 | 13 |
| 14 | Dp 116-20 | cAuuTucAgcTugAacCg | 1836358 | 1836375 | 6288.67 | 14 |

In the sequences shown in the Table, capital letters indicate that D-ribofuranose is 2'-O,4'-C-ethylenated, and small letters indicate that D-ribofuranose is 2'-O-methylated. All the bonds between nucleosides are a phosphorothioate bond. Briefly, capital letters represent any one of A^{e2s}, G^{e2s}, C^{e2s} or T^{e2s}; small letters other than those located at the 3' end represent any one of A^{mls}, G^{mls}, C^{mls} or U^{mls}; and small letters located at 3' end represent any one of A^{mlt}, G^{mlt}, C^{mlt} or U^{mlt}. For "Start" and "End", respective nucleotide numbers in Homo sapiens dystrophin (DMD), RefSeqGene (LRG_199) on chromosome X (NCBI-GenBank accession No. NG_012232.1) are shown. The sequences in the Table show those which are complementary to the respective nucleotide sequences from "Start" to "End". Molecular weights in the Table show values as measured by negative-ion ESI mass spectrometry.

### [Examples 1 to 10]

The compounds of Examples 1 to 10 were also synthesized in the same manner as in Reference Example 1. Data from Examples 1 to 10 are summarized in Table 2 below.

**Table 2**

| Example | Designation | Sequence (5'-3') | Start | End | Molecular Weight | SEQ ID NO: |
|---|---|---|---|---|---|---|
| 1 | Dp 116-08 | uAcaTggTauGucTucCu | 1836382 | 1836399 | 6280.65 | 15 |
| 2 | Dp 116-09 | aCauGguAugTcuTccTg | 1836381 | 1836398 | 6319.66 | 16 |
| 3 | Dp116-10 | cAugGuaTguCuuCcuGu | 1836380 | 1836397 | 6282.62 | 17 |
| 4 | Dp116-11 | aTggTauGucTucCugTg | 1836379 | 1836396 | 6350.66 | 18 |
| 5 | Dp116-12 | uGguAugTcuTccTguGu | 1836378 | 1836395 | 6299.60 | 19 |
| 6 | Dp116-13 | gGuaTguCuuCcuGugTa | 1836377 | 1836394 | 6336.63 | 20 |
| 7 | Dp116-14 | gTauGucTucCugTguAa | 1836376 | 1836393 | 6320.64 | 21 |
| 8 | Dp116-15 | uGucTucCugTguAacAu | 1836373 | 1836390 | 6266.66 | 22 |
| 9 | Dp 116-21 | uTucAgcTugAacCggGc | 1836355 | 1836372 | 6343.69 | 23 |
| 10 | Dp 116-22 | cAgcTugAacCggGcaCu | 1836352 | 1836369 | 6365.75 | 24 |

In the sequences shown in the Table, capital letters indicate that D-ribofuranose is 2'-O,4'-C-ethylenated, and small letters indicate that D-ribofuranose is 2'-O-methylated. All the bonds between nucleosides are a phosphorothioate bond. Briefly, capital letters represent any one of A^{e2s}, G^{e2s}, C^{e2s} or T^{e2s}; small letters other than those located at the 3' end represent any one of A^{mls}, G^{mls}, C^{mls} or U^{mls}; and small letters located at 3' end represent any one of A^{mlt}, G^{mlt}, C^{mlt} or U^{mlt}. For "Start" and "End", the respective nucleotide numbers in Homo sapiens dystrophin (DMD), RefSeqGene (LRG_199) on chromosome X (NCBI-GenBank accession No. NG_012232.1) are shown. The sequences in the Table show those which are complementary to the respective nucleotide sequences from "Start" to "End". Molecular weights in the Table show values as measured by negative-ion ESI mass spectrometry.

### [Examples 11 to 18]

The compounds of Examples 11 to 18 were also synthesized in the same manner as in Reference Example 1. Data from Examples 11 to 18 are summarized in Table 3 below.

**Table 3**

| Example | Designation | Sequence (5'-3') | Start | End | Molecular Weight | SEQ ID NO: |
|---|---|---|---|---|---|---|
| 11 | Dp116-13a | ggTaTgucuTccTgTgTa | 1836377 | 1836394 | 6364.70 | 25 |
| 12 | Dp116-13b | ggTaTgucTuccTgTgTa | 1836377 | 1836394 | 6364.70 | 26 |
| 13 | Dp116-13c | ggTaTguCuucCugTgTa | 1836377 | 1836394 | 6364.68 | 27 |
| 14 | Dp116-13d | ggTaTguCuuCcugTgTa | 1836377 | 1836394 | 6364.70 | 28 |
| 15 | Dp116-13e | ggTaTgTcuTccTgTgTa | 1836377 | 1836394 | 6390.71 | 29 |
| 16 | Dp116-13f | ggTaTgTcTuccTgTgTa | 1836377 | 1836394 | 6390.69 | 30 |
| 17 | Dp116-13g | ggTaTgTcTTccTgTgTa | 1836377 | 1836394 | 6416.72 | 31 |
| 18 | Dp116-13h | ggTaTgTcTuCcTgTgTa | 1836377 | 1836394 | 6416.72 | 32 |

In the sequences shown in the Table, capital letters indicate that D-ribofuranose is 2'-O,4'-C-ethylenated, and small letters indicate that D-ribofuranose is 2'-O-methylated. All the bonds between nucleosides are a phosphorothioate bond. Briefly, capital letters represent any one of A^{e2s}, G^{e2s}, C^{e2s} or T^{e2s}; and small letters represent any one of A^{mls}, G^{mls}, C^{mls}, U^{mls}, A^{mlt}, G^{mlt}, C^{mlt} or U^{mlt}. For "Start" and "End", the respective nucleotide numbers in Homo sapiens dystrophin (DMD), RefSeqGene (LRG_199) on chromosome X (NCBI-GenBank accession No. NG_012232.1) are shown. The sequences in the Table show those which are complementary to the respective nucleotide sequences from "Start" to "End". Molecular weights in the Table show values as measured by negative-ion ESI mass spectrometry.

### [Examples 19 to 45]

The compounds of Examples 19 to 45 were also synthesized in the same manner as in Reference Example 1. Data from Examples 19 to 45 are summarized in Table 4 below.

**Table 4**

| Example | Designation | Sequence (5'-3') | Start | End | Molecular Weight | SEQ ID NO: |
|---|---|---|---|---|---|---|
| 19 | Dp116-13a.1 | ggTaTgucuTccTgTgT | 1836378 | 1836394 | 6005.6455 | 33 |
| 20 | Dp116-13a.2 | gTaTgucTuccTgTgTa | 1836377 | 1836393 | 5989.6571 | 34 |
| 21 | Dp116-13a.3 | ggTaTguCuucCugTg | 1836379 | 1836394 | 5643.6161 | 35 |
| 22 | Dp116-13a.4 | gTaTguCuuCcugTgT | 1836378 | 1836395 | 5630.6015 | 36 |
| 23 | Dp116-13a.5 | TaTgTcuTccTgTgTa | 1836377 | 1836392 | 5614.6060 | 37 |
| 24 | Dp116-13a.6 | ggTaTgTcTuccTgT | 1836374 | 1836394 | 5268.5651 | 38 |
| 25 | Dp116-13a.7 | gTaTgTcTTccTgTg | 1836375 | 1836393 | 5268.5675 | 39 |
| 26 | Dp116-13a.8 | TaTgTcTuCcTgTgT | 1836376 | 1836392 | 5255.5722 | 40 |
| 27 | Dp116-13a.9 | aTgucuTccTgTgTa | 1836377 | 1836391 | 5252.5687 | 41 |
| 28 | Dp116-13e.1 | ggTaTgucuTccTgTgT | 1836378 | 1836394 | 6031.6439 | 42 |
| 29 | Dp116-13e.2 | gTaTgucTuccTgTgTa | 1836377 | 1836393 | 6015.6527 | 43 |
| 30 | Dp116-13e.3 | ggTaTguCuucCugTg | 1836379 | 1836394 | 5669.6344 | 44 |
| 31 | Dp116-13e.4 | gTaTguCuuCcugTgT | 1836378 | 1836395 | 5656.6177 | 45 |
| 32 | Dp116-13e.5 | TaTgTcuTccTgTgTa | 1836377 | 1836392 | 5640.6201 | 46 |
| 33 | Dp116-13e.6 | ggTaTgTcTuccTgT | 1836374 | 1836394 | 5294.5832 | 47 |
| 34 | Dp116-13e.7 | gTaTgTcTTccTgTg | 1836375 | 1836393 | 5294.5835 | 48 |
| 35 | Dp116-13e.8 | TaTgTcTuCcTgTgT | 1836376 | 1836392 | 5281.5756 | 49 |
| 36 | Dp116-13e.9 | aTgucuTccTgTgTa | 1836377 | 1836391 | 5278.5856 | 50 |
| 37 | Dp116-13g.1 | ggTaTgucuTccTgTgT | 1836378 | 1836394 | 6057.6588 | 51 |
| 38 | Dp116-13g.2 | gTaTgucTuccTgTgTa | 1836377 | 1836393 | 6041.6637 | 52 |
| 39 | Dp116-13g.3 | ggTaTguCuucCugTg | 1836379 | 1836394 | 5695.6337 | 53 |
| 40 | Dp116-13g.4 | gTaTguCuuCcugTgT | 1836378 | 1836395 | 5682.6421 | 54 |
| 41 | Dp116-13g.5 | TaTgTcuTccTgTgTa | 1836377 | 1836392 | 5666.6295 | 55 |
| 42 | Dp116-13g.6 | ggTaTgTcTuccTgT | 1836374 | 1836394 | 5320.5994 | 56 |
| 43 | Dp116-13g.7 | gTaTgTcTTccTgTg | 1836375 | 1836393 | 5320.5993 | 57 |
| 44 | Dp116-13g.8 | TaTgTcTuCcTgTgT | 1836376 | 1836392 | 5307.5983 | 58 |
| 45 | Dp116-13g.9 | aTgucuTccTgTgTa | 1836377 | 1836391 | 5304.6046 | 59 |

In the sequences shown in the Table, capital letters indicate that D-ribofuranose is 2'-O,4'-C-ethylenated, and small letters indicate that D-ribofuranose is 2'-O-methylated. All the bonds between nucleosides are a phosphorothioate bond. Briefly, capital letters represent any one of A^{e2s}, G^{e2s}, C^{e2s} or T^{e2s}; and small letters represent any one of A^{mls}, G^{mls}, C^{mls}, U^{mls}, A^{mlt}, G^{mlt}, C^{mlt} or U^{mlt}. For "Start" and "End", the respective nucleotide numbers in Homo sapiens dystrophin (DMD), RefSeqGene (LRG_199) on chromosome X (NCBI-GenBank accession No. NG_012232.1) are shown. The sequences in the Table show those which are complementary to the respective nucleotide sequences from "Start" to "End". Molecular weights in the Table show values as measured by negative-ion ESI mass spectrometry.

### [Test Example 1]

Suppression of Dp116 Expression in U251 Cells by the Compounds of Examples and Reference Examples

Glioblastoma-derived U251 cells were purchased from ATCC. The cells were cultured at 37°C under 5% CO₂ in air.

### Transfection of U251 Cells with Oligonucleotides

U251 cells were transfected as described below with the compounds (antisense oligonucleotides) prepared in Examples and Reference Examples.
1. Each of the compounds prepared in Examples (adjusted to a concentration of 10 µg/20 µl with Milli-Q) (200 pmol) was dissolved in 100 µl of Opti-MEM (GIBCO-BRL).
2. To the solution prepared in 1 above, 6 µl of plus reagent (GIBCO-BRL) was added and the resultant solution was left at room temperature for 15 min.
3. Using a separate tube, 8 µl of Lipofectamine (GIBCO-BRL) was dissolved in 100 µl of Opti-MEM.
4. After the treatment in 2 above, the solution of 3 was added to the treated solution, which was left at room temperature for another 15 min.
5. Myoblasts 4 days after directed differentiation were washed once with PBS, followed by addition of 800 µl of Opti-MEM.
6. After the treatment in 4 above, the treated solution was added to the cells of 5 above.
7. The cells of 6 above were cultured at 37°C under 5% CO₂ in air for 3 hrs, and then 500 µl of DMEM (containing 6% HS) was added to each well.
8. Culture was further continued.

### RNA Extraction

RNA extraction was performed as described below.
1. Antisense oligonucleotide-transfected cells were cultured for one day and then washed with PBS once. Subsequently, ISOGEN (Nippon Gene) (500 µl) was added to the cells.
2. The cells were left at room temperature for 5 min and, thereafter, ISOGEN in each well was collected into a tube.
3. RNA was extracted according to the protocol of ISOGEN (Nippon Gene).
4. Finally, RNA was dissolved in 20 µl of DEPW.

### Reverse Transcription Reaction

Reverse transcription reaction was performed as described below.
1. DEPW (sterile water treated with diethylpyrocarbonate) was added to 2 µg of RNA to prepare a 6 µl solution.
2. To the solution of 1 above, 2 µl of random hexamer (20-fold dilution of Invitrogen 3 µg/µl) was added.
3. The solution of 2 above was heated at 65°C for 10 min.
4. The solution of 3 above was cooled on ice for 2 min.
5. To the above reaction solution, 1 µl of MMLV-reverse transcriptase (Invitrogen 200 U/µl), 1 µl of Human placenta ribonuclease inhibitor (Takara 40 U/µl), 1 µl of DTT (attached to MMLV-reverse transcriptase), 4 µl of buffer (attached to MMLV-reverse transcriptase), and 5 µl of dNTPs (attached to Takara Ex Taq) were added.
6. The resultant reaction mixture was kept warm at 37°C for 1 hr and then heated at 95°C for 5 min.
7. After the reaction, the reaction mixture was stored at -80°C.

### PCR Reaction

PCR was performed as described below.
1. The components listed below were mixed and then heated at 94°C for 4 min.

| | |
|---|---|
| Reverse transcription reaction product | 3 µl |
| Forward primer (10 pmol/µl) | 1 µl |
| Reverse primer (10 pmol/µl) | 1 µl |
| dNTP (attached to TAKARA Ex Taq) | 2 µl |
| Buffer (attached to TAKARA Ex Taq) | 2 µl |
| Ex Taq (TAKARA) | 0.1 µl |
| Sterile water | 11 µl |

2. After the treatment at 94°C for 4 min, a treatment consisting of 94°C 1 min, 60°C 1 min and 72°C 3 min was performed through 35 cycles.
3. Finally, the reaction mixture was heated at 72°C for 7 min.
Nucleotide sequences of the forward and reverse primers used in the PCR for detecting suppression of Dp166 expression were as follows.
Forward primer Dp116ex1F-2: 5'- GGGTTTTCTCAGGATTGCTATGC -3' (SEQ ID NO: 60)
Reverse primer 4F: 5'- CCCACTCAGTATTGACCTCCTC -3' (SEQ ID NO: 61)

As an internal standard, the *GAPDH* gene was used. Nucleotide sequences of the forward and reverse primers used in PCR were as follows.
Forward primer GAPDH-F: 5'- CCCTTCATTGACCTCAAC -3' (SEQ ID NO: 62)
Reverse primer GAPDH-R: 5'- TTCACACCCATGACGAAC -3' (SEQ ID NO: 63)

4. Analyses of PCR products were performed with Agilent Bioanalyzer. After electrophoretic separation, the amount of each band was quantitatively determined.
5. Sequencing of PCR products

Amplified products were analyzed by 2% agarose gel electrophoresis.

Bands of amplified products were cut out from the gel. The PCR product was subcloned into pT7 Blue-T vector (Novagen) and confirmed for its nucleotide sequence by sequencing on ABI PRISM 310 Genetic Analyzer (Applied Biosystems) with Thermo Sequenqse™ II dye terminator cycle sequencing kit (Amersham Pharmacia Biotec). Reaction procedures were according to the attached manual.

### [Results]

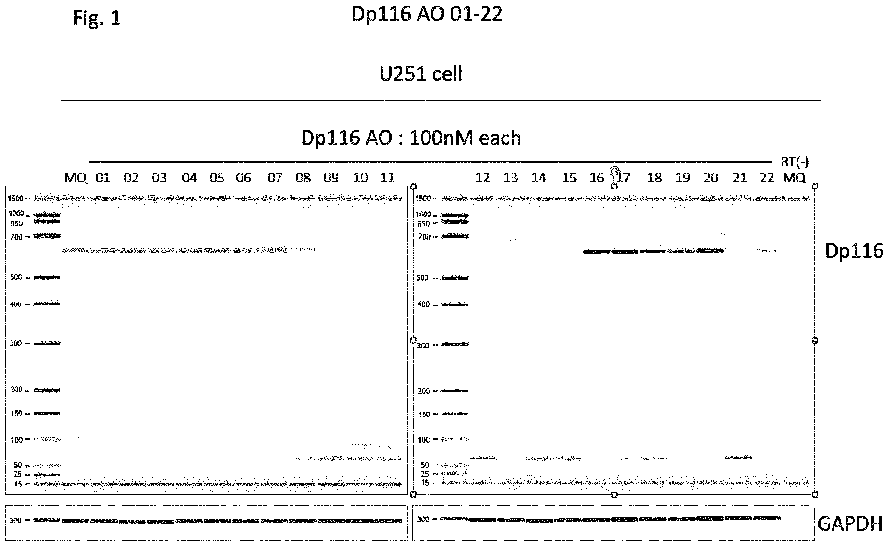

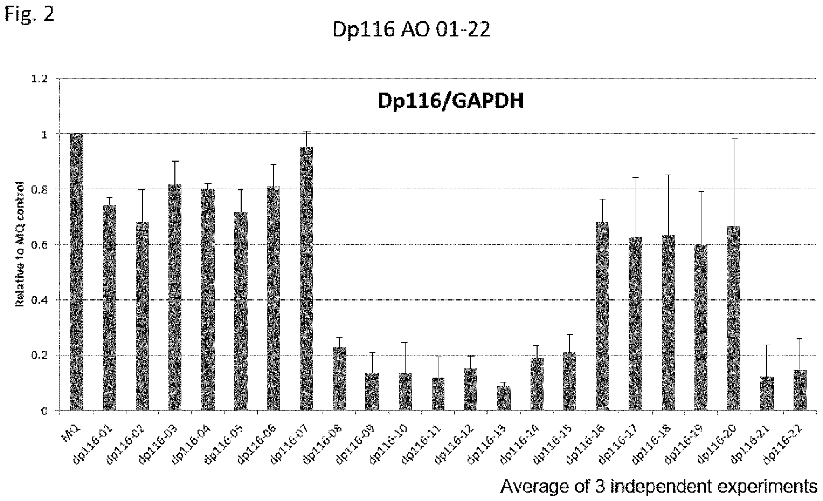

Antisense oligonucleotides Dp116-01 to Dp116-022 prepared in Examples and Reference Examples were introduced into U251 cells and, after 24 hrs, mRNAs were analyzed by RT-PCR. From the cells into which only MQ (Milli-Q water) was introduced, amplified products were obtained. Likewise, Dp116 was amplified by RT-PCR using RNAs extracted from the cells transfected with various antisense oligonucleotides. Bands of amplified products were obtained at varying densities. (In Fig. 1, lane numbers 01 to 22 represent Dp116-01 to Dp116-022, respectively. The lane MQ means that MQ was used instead of antisense oligonucleotide.) Then, semi-quantitative analysis of these amplified products was performed. The results revealed that Dp 116-08 to Dp 116-015, Dp 116-021 and Dp 116-022 lowered the Dp116 mRNA level to approximately 20% or even less, compared to the control. Especially, Dp 116-13 lowered the Dp116 level by the greatest degree. The Dp116 mRNA level was found to decrease to approximately 10% of the level in the cells using MQ (Fig. 2).

From these results, the present inventors determined that Dp116-13 is the most promising antisense oligonucleotide capable of suppressing Dp116 expression.

### [Test Example 2]

### Suppression of Dp116 Protein Expression in MiraCell Cardiomyocytes by the Compounds of Examples

To 96-well plates, 0.1 µg/mL of fibronectin solution (F1141-1MG, SIGMA) or 0.1% gelatin solution (190-15805, FUJIFILM Wako Pure Chemical Corporation) was added in an amount of 100 µl per well and incubation was conducted at 37°C for 2 hr to thereby coat the plates. The solution remaining in the well was removed. At the same time, MiraCell cardiomyocytes (Y50015, Takara Bio) thawed according to the procedures written in the attached instructions and suspended in Thawing Medium (Y50015, Takara Bio) were seeded on 96-well plates at 1x10⁵ cells/well in a volume of 100 µl. The cells were cultured at 37°C in a 5% CO₂ incubator for 2 days, and then the medium was replaced with Culture Medium (Y50013, Takara Bio). After culture for another day, 1 µM of an Example compound (Dp116-13, Dp116-13a or Dp116-13e) suspended in OptiMEM (31985062, GIBCO) and 5% Lipofectamine 2000 (52887, Invitrogen) suspended in OptiMEM were mixed at a ratio of 1:1 and incubated for 20 min. The resultant solution was added in an amount of 10 µl per well to thereby effect transfection. As a negative control, Lipofectamine 2000 solution alone was added. As a positive control, a Dp116 expressing plasmid was added. After 3 day culture (at day 6), the cells were washed with PBS (10010023, GIBCO) twice. A lysis buffer [125 mM Tris-HCl (pH6.8), 4% SDS, 4M urea, supplemented with protease inhibitor (P8340, SIGMA)] was added in an amount of 50 µl per well to lyse cells, whereby a lysate was prepared.

A sample buffer [Laemmli sample buffer (1610737, Bio-Rad) supplemented with 100 mM DTT] and the lysate were mixed 1:1 in volume and heated at 95°C for 5 min. The resultant sample was applied to 4-20% Criterion TGX precast gel (5671095, Bio-Rad) in 10 µl portions per well and electrophoresed at 150 V for 60 min. After electrophoresis, proteins on the gel were transferred onto a PVDF membrane using iBlot2 dry blotting system (Thermo Scientific). After blocking with StartingBlock (TBS) Blocking Buffer (37579, Thermo Scientific), reaction was performed overnight at 4°C with anti-dystrophin antibody (ab 15277, abcam) diluted 1000-fold with Can Get Signal Immunoreaction Enhancer solution 1 (NKB-101, TOYOBO). After washing with TBS-T (9997S, CST), reaction was performed at room temperature for 1 hr with Anti-rabbit IgG, HRP-linked whole Ab Donkey (NA-934-1mL, GE Healthcare) diluted 20000-fold with Can Get Signal solution 2. After washing the PVDF membrane with TBS-T, reaction was performed with Luminata Forte Western HRP substrate (WBLUF0500, Merck-Millipore) for 1 min and the chemiluminescent bands were detected with ImageQuant LAS4000 (GE Healthcare). With respect to a loading control β-actin, the PVDF membrane after Dp116 detection was soaked in Restore PLUS Western Blot Stripping Buffer (46430, Thermo Scientific) at room temperature for 15 min to effect stripping, then reacted with anti-β-actin antibody (4970S, CST) diluted 1000-fold with Can Get Signal Immunoreaction Enhancer solution 1 and with Anti-rabbit IgG, HRP-linked whole Ab Donkey diluted 20000-fold with Can Get Signal solution 2, each reaction being conducted at room temperature for 1; upon reaction with Luminata Forte Western HRP substrate, the chemiluminescent bands were detected with ImageQuant LAS4000.

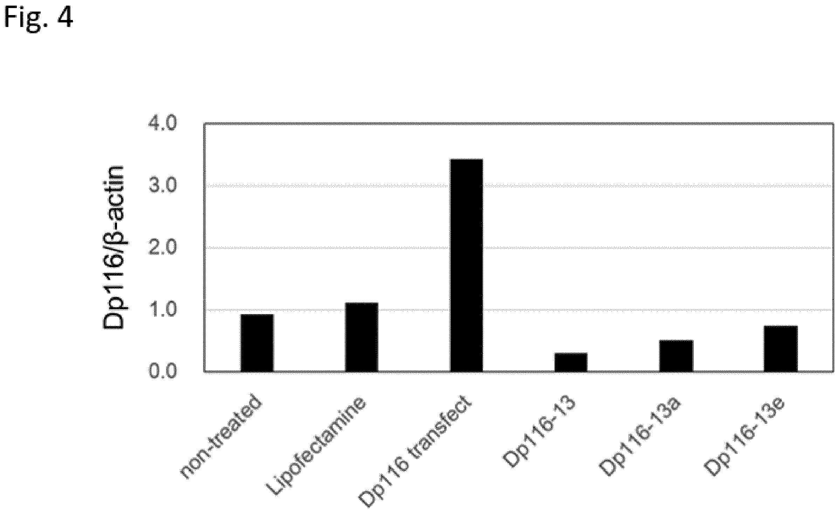

As regards bands of Dp116 and β-actin, ImageQuant TL software was used to calculate values that were the average band emission intensities of respective bands minus the background emission intensity. For correcting the amount of load, the band intensity of Dp116 was divided by that of β-actin and the respective values were shown in a graph (Fig. 4).

The band intensity of Dp116 protein increased in the cells transfected with Dp116 plasmid (positive control). On the other hand, in the cells transfected with Example compounds (Dp116-13, Dp116-13a and Dp116-13e), the band intensity decreased as compared to the control, i.e., the expression level of Dp116 protein decreased. Notably, Dp116-13 decreased the expression level of Dp116 protein to approximately 30%, relative to the control.

### [Test Example 3]

### Suppression of Dp116 Expression in U251 Cells by Compounds of Examples

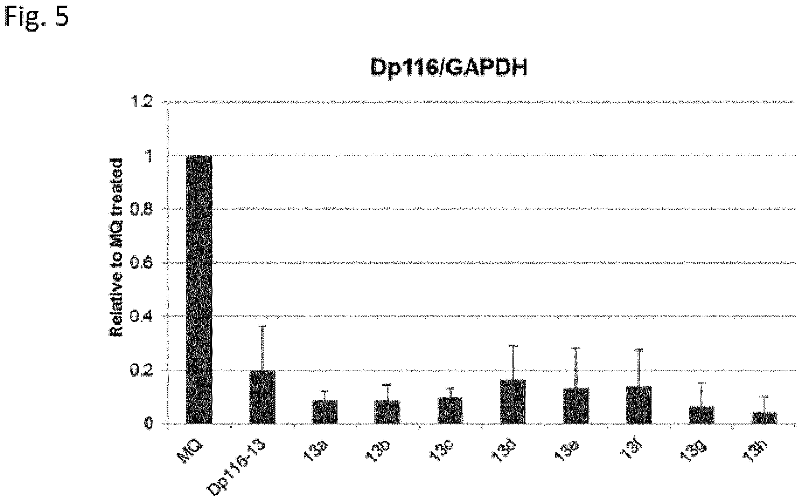

Example compounds (Dp116-13 and Dp116-13a to Dp116-13h) were evaluated by the same methods as in Test Example 1. Consequently, Dp116-13 and Dp116-13a to Dp116-13h decreased the Dp116 mRNA level to approximately 20% or less, relative to the control (Fig. 5). Notably, Dp116-13a, -13b, -13c, -13g and -13h decreased the Dp116 mRNA level to approximately 10% or even less, relative to the control.

### [Test Example 4]

### Suppression of Dp116 Expression in U251 Cells by Compounds of Examples

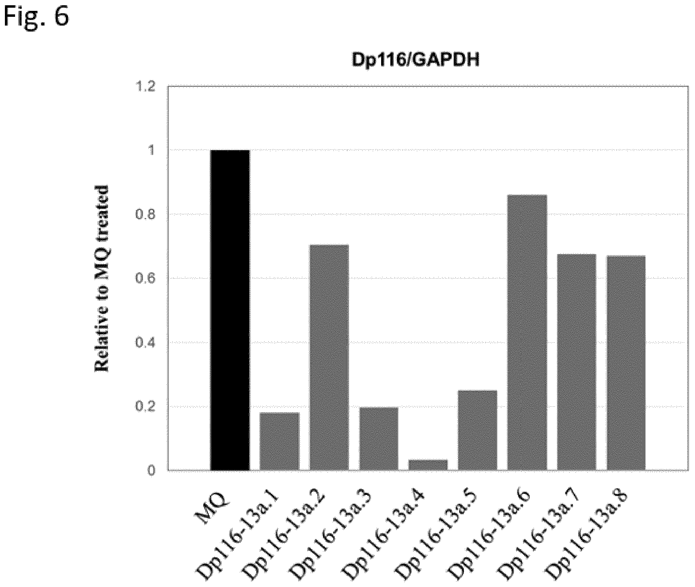

Example compounds (Dp116-13a.1 to Dp116-13a.8) were evaluated by the same methods as in Test Example 1. Consequently, Dp116-13a.1 to Dp116-13a.8 decreased the Dp116 mRNA level, relative to the control (Fig. 6). Notably, Dp116-13a.1 and Dp116-13a.3 to Dp116-13a.5 decreased the Dp116 mRNA level to approximately 20% or even less, relative to the control.

All publications, patents and patent applications cited herein are incorporated herein by reference in their entirety.

### INDUSTRIAL APPLICABILITY

The present invention is applicable to prevention and/or treatment of cardiac dysfunction.

### SEQUENCE LISTING FREE TEXT

<SEQ ID NOS: 1 to 59>
   These sequences show the nucleotide sequences of antisense oligonucleotides. Nucleotides constituting the antisense oligonucleotides may be either natural DNA, natural RNA, chimera DNA/RNA, or modified DNA, RNA or DNA/RNA, with at least one of these being optionally a modified nucleotide.
<SEQ ID NOS: 60 to 63>
   These sequences show primer sequences.
<SEQ ID NO: 64>
   This shows the sequence of antisense oligonucleotide. Nucleotides constituting the antisense oligonucleotide may be either natural DNA, natural RNA, chimera DNA/RNA, or modified DNA, RNA or DNA/RNA, with at least one of these being optionally a modified nucleotide.

## Claims

1. An oligonucleotide, a pharmacologically acceptable salt thereof, or a solvate thereof, wherein the oligonucleotide having 15-30 base comprises a nucleotide sequence complementary to a part of the intron 55 region of a dystrophin gene, and comprises the sequence of 5'-TGTCTTCCT-3' or 5'-CAGCTTGAACCGGGC-3' (SEQ ID NO: 64) (wherein "T" may be "U" in either sequence).

2. The oligonucleotide, a pharmacologically acceptable salt thereof of claim 1, which comprises the sequence of 5'-TGTCTTCCT-3' (wherein "T" may be "U").

3. The oligonucleotide, a pharmacologically acceptable salt thereof, or a solvate thereof of claim 1 or 2, which comprises any one of the sequences of SEQ ID NOS: 15 to 59 (wherein "T" may be "U", and "U" may be "T").

4. The oligonucleotide, a pharmacologically acceptable salt thereof, or a solvate thereof of claim 1 or 2, which comprises any one of the sequences of SEQ ID NOS: 20, 25 to 33 and 35 to 37.

5. The oligonucleotide, a pharmacologically acceptable salt thereof, or a solvate thereof of any one of claims 1 to 4, which is capable of suppressing the expression of dystrophin Dp116.

6. The oligonucleotide, a pharmacologically acceptable salt thereof, or a solvate thereof of any one of claims 1 to 5, wherein at least one of the sugar and/or the phosphodiester bond constituting the oligonucleotide is modified.

7. The oligonucleotide, a pharmacologically acceptable salt thereof, or a solvate thereof of any one of claims 1 to 6, wherein the sugar constituting the oligonucleotide is D-ribofuranose and modification of the sugar is modification of the hydroxy group at 2'-position of D-ribofuranose.

8. The oligonucleotide, a pharmacologically acceptable salt thereof, or a solvate thereof of any one of claims 1 to 7, wherein the sugar constituting the oligonucleotide is D-ribofuranose and modification of the sugar is 2'-O-alkylation and/or 2'-,4'-bridge of D-ribofuranose.

9. The oligonucleotide, a pharmacologically acceptable salt thereof, or a solvate thereof of any one of claims 1 to 8, wherein the sugar constituting the oligonucleotide is D-ribofuranose and modification of the sugar is 2'-O-alkylation and/or 2'-O,4'-C-alkylenation of D-ribofuranose.

10. The oligonucleotide, a pharmacologically acceptable salt thereof, or a solvate thereof of any one of claims 1 to 8, wherein the sugar constituting the oligonucleotide is D-ribofuranose and modification of the sugar is 2'-O-methylation and/or 2'-O,4'-C-ethylenation of D-ribofuranose.

11. The oligonucleotide, a pharmacologically acceptable salt thereof, or a solvate thereof of any one of claims 1 to 10, wherein modification of the phosphodiester bond constituting the oligonucleotide is a phosphorothioate bond.

12. A prophylactic and/or a therapeutic agent for cardiac dysfunction, comprising the oligonucleotide, a pharmacologically acceptable salt thereof, or a solvate thereof of any one of claims 1 to 11.

13. The prophylactic and/or therapeutic agent of claim 12, which is to be applied to patients expressing dystrophin Dp116.

14. The prophylactic and/or therapeutic agent of claim 13, wherein the patients expressing dystrophin Dp116 are patients with Duchenne muscular dystrophy.

15. A suppressor of Dp116 expression, comprising the oligonucleotide, a pharmacologically acceptable salt thereof, or a solvate thereof of any one of claims 1 to 11.

16. A method of preventing and/or treating cardiac dysfunction, comprising administering to a subject a pharmacologically effective amount of the oligonucleotide, a pharmacologically acceptable salt thereof, or a solvate thereof of any one of claims 1 to 11.

17. A method of suppressing the expression of Dp116, comprising treating a Dp116 expressing cell, tissue or organ with the oligonucleotide, a pharmacologically acceptable salt thereof, or a solvate thereof of any one of claims 1 to 11.

18. The oligonucleotide, a pharmacologically acceptable salt thereof, or a solvate thereof of any one of claims 1 to 11, for use in a method of preventing and/or treating cardiac dysfunction.

19. Use of the oligonucleotide, a pharmacologically acceptable salt thereof, or a solvate thereof of any one of claims 1 to 11, for suppressing the expression of Dp116.

20. A formulation for oral or parenteral administration, comprising the oligonucleotide, a pharmacologically acceptable salt thereof, or a solvate thereof of any one of claims 1 to 11.

21. The oligonucleotide, a pharmacologically acceptable salt thereof, or a solvate thereof of any one of claims 1 to 11, for use as a pharmaceutical drug.


SEQUENCE LISTING

<110>  DAIICHI SANKYO COMPANY, LIMITED
       KOBE GAKUIN UNIVERSITY

<120>  Therapeutic agents against myocardial disorder

<130>  FP-262PCT

<150>  JP2018-112863
<151>  2018-06-13

<160>  64

<170>  PatentIn version 3.5

<210>  1
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  1
atagtagaag aaucugac                                                    18


<210>  2
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  2
gtagaagaau cugacctu                                                    18


<210>  3
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  3
gaagaaucug acctuuac                                                    18


<210>  4
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  4
gaaucugacc tuuacatg                                                    18


<210>  5
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  5
ucugacctuu acatggta                                                    18


<210>  6
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  6
gacctuuaca tggtaugu                                                    18


<210>  7
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  7
   ctuuacatgg taugucta                                                     18


<210>  8
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  8
utuacauggu augtcutc                                                    18


<210>  9
<211>  18
<212>  DNA
<213>  Artificial Sequence

<220>

<223>  Antisense oligonucleotide which is a DNA, RNA or DNA/RNA chimeric
       molecule, with at least one nucleotide being optionally modified

<400>  9
utacauggua tgucuucc                                                    18


<210>  10
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  10
ctuccugtgu aacauutu                                                    18


<210>  11
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  11
ccugtguaac auutucag                                                    18


<210>  12
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  12
gtguaacauu tucagctu                                                    18


<210>  13
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  13
uaacauutuc agctugaa                                                    18


<210>  14
<211>  18
<212>  DNA
<213>  Artificial Sequence

<220>
<223>  Antisense oligonucleotide which is a DNA, RNA or DNA/RNA chimeric
      molecule, with at least one nucleotide being optionally modified

<400>  14
cauutucagc tugaaccg                                                    18


<210>  15
<211>  18
<212>  DNA
<213>  Artificial Sequence

<220>
<223>  Antisense oligonucleotide which is a DNA, RNA or DNA/RNA chimeric
      molecule, with at least one nucleotide being optionally modified

<400>  15
uacatggtau gucuuccu                                                    18


<210>  16
<211>  18
<212>  DNA
<213>  Artificial Sequence

<220>
<223>  Antisense oligonucleotide which is a DNA, RNA or DNA/RNA chimeric
      molecule, with at least one nucleotide being optionally modified

<400>  16
acaugguaug tcutcctg                                                    18


<210>  17
<211>  18
<212>  DNA
<213>  Artificial Sequence

<220>
<223>  Antisense oligonucleotide which is a DNA, RNA or DNA/RNA chimeric
      molecule, with at least one nucleotide being optionally modified

<400>  17
caugguatgu cuuccugu                                                    18


<210>  18
<211>  18
<212>  DNA
<213>  Artificial Sequence

<220>
<223>  Antisense oligonucleotide which is a DNA, RNA or DNA/RNA chimeric
      molecule, with at least one nucleotide being optionally modified

<400>  18
atggtauguc tuccugtg                                                    18


<210>  19
<211>  18

<212>  DNA
<213>  Artificial Sequence

<220>
<223>  Antisense oligonucleotide which is a DNA, RNA or DNA/RNA chimeric
       molecule, with at least one nucleotide being optionally modified

<400>  19
ugguaugtcu tcctgugu                                                    18


<210>  20
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  20
gguatgucuu ccugugta                                                    18


<210>  21
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  21
gtauguctuc cugtguaa                                                    18


<210>  22
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  22
uguctuccug tguaacau                                                    18


<210>  23
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  23
utucagctug aaccgggc                                                    18

<210>  24
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  24
cagctugaac cgggcacu                                                    18


<210>  25
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  25
ggtatgucut cctgtgta                                                    18


<210>  26
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  26
ggtatguctu cctgtgta                                                    18


<210>  27
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  27
ggtatgucuu ccugtgta                                                    18


<210>  28
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  28
ggtatgucuu ccugtgta                                                    18

<210>  29
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  29
ggtatgtcut cctgtgta                                                    18


<210>  30
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  30
ggtatgtctu cctgtgta                                                    18


<210>  31
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  31
ggtatgtctt cctgtgta                                                    18


<210>  32
<211>  18
<212>  DNA
<213>  Artificial Sequence

<220>
<223>  Antisense oligonucleotide which is a DNA, RNA or DNA/RNA chimeric
       molecule, with at least one nucleotide being optionally modified

<400>  32
ggtatgtctu cctgtgta                                                    18


<210>  33
<211>  17
<212>  DNA
<213>  Artificial Sequence

<220>
<223>  Antisense oligonucleotide which is a DNA, RNA or DNA/RNA chimeric
       molecule, with at least one nucleotide being optionally modified

<400>  33
ggtatgucut cctgtgt                                                    17


<210>  34
<211>  17
<212>  DNA
<213>  Artificial Sequence

<220>
<223>  Antisense oligonucleotide which is a DNA, RNA or DNA/RNA chimeric
       molecule, with at least one nucleotide being optionally modified

<400>  34
   gtatguctuc ctgtgta                                                   17


<210>  35
<211>  16
<212>  DNA
<213>  Artificial Sequence

<220>
<223>  Antisense oligonucleotide which is a DNA, RNA or DNA/RNA chimeric
       molecule, with at least one nucleotide being optionally modified

<400>  35
ggtatgucuu ccugtg                                                     16


<210>  36
<211>  16
<212>  DNA
<213>  Artificial Sequence

<220>
<223>  Antisense oligonucleotide which is a DNA, RNA or DNA/RNA chimeric
       molecule, with at least one nucleotide being optionally modified

<400>  36
gtatgucuuc cugtgt                                                     16


<210>  37
<211>  16
<212>  DNA
<213>  Artificial Sequence

<220>
<223>  Antisense oligonucleotide which is a DNA, RNA or DNA/RNA chimeric
       molecule, with at least one nucleotide being optionally modified

<400>  37
tatgtcutcc tgtgta                                                     16


<210>  38
<211>  15
<212>  DNA
<213>  Artificial Sequence

<220>
<223>  Antisense oligonucleotide which is a DNA, RNA or DNA/RNA chimeric

molecule, with at least one nucleotide being optionally modified

<400>  38
ggtatgtctu cctgt                                                        15


<210>  39
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  39
gtatgtcttc ctgtg                                                        15


<210>  40
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  40
tatgtctucc tgtgt                                                        15


<210>  41
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  41
atgucutcct gtgta                                                        15


<210>  42
<211>  17
<212>  DNA
<213>  Artificial Sequence

<220>
<223>  Antisense oligonucleotide which is a DNA, RNA or DNA/RNA chimeric
       molecule, with at least one nucleotide being optionally modified

<400>  42
ggtatgucut cctgtgt                                                      17


<210>  43
<211>  17
<212>  DNA
<213>  Artificial Sequence

<220>
<223>  Antisense oligonucleotide which is a DNA, RNA or DNA/RNA chimeric
      molecule, with at least one nucleotide being optionally modified

<400>  43
gtatgucuuc ctgtgta                                                    17


<210>  44
<211>  16
<212>  DNA
<213>  Artificial Sequence

<220>
<223>  Antisense oligonucleotide which is a DNA, RNA or DNA/RNA chimeric
      molecule, with at least one nucleotide being optionally modified

<400>  44
ggtatgucuu ccugtg                                                     16


<210>  45
<211>  16
<212>  DNA
<213>  Artificial Sequence

<220>
<223>  Antisense oligonucleotide which is a DNA, RNA or DNA/RNA chimeric
      molecule, with at least one nucleotide being optionally modified

<400>  45
gtatgucuuc cugtgt                                                     16


<210>  46
<211>  16
<212>  DNA
<213>  Artificial Sequence

<220>
<223>  Antisense oligonucleotide which is a DNA, RNA or DNA/RNA chimeric
      molecule, with at least one nucleotide being optionally modified

<400>  46
tatgtcutcc tgtgta                                                     16


<210>  47
<211>  15
<212>  DNA
<213>  Artificial Sequence

<220>
<223>  Antisense oligonucleotide which is a DNA, RNA or DNA/RNA chimeric
      molecule, with at least one nucleotide being optionally modified

<400>  47
ggtatgtctu cctgt                                                      15


<210>  48
<211>  15
<212>  DNA

<213>  Artificial Sequence

<220>
<223>  Antisense oligonucleotide which is a DNA, RNA or DNA/RNA chimeric
       molecule, with at least one nucleotide being optionally modified

<400>  48
gtatgtcttc ctgtg                                                        15


<210>  49
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  49
tatgtctucc tgtgt                                                        15


<210>  50
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  50
atgucutcct gtgta                                                        15


<210>  51
<211>  17
<212>  DNA
<213>  Artificial Sequence

<220>
<223>  Antisense oligonucleotide which is a DNA, RNA or DNA/RNA chimeric
       molecule, with at least one nucleotide being optionally modified

<400>  51
ggtatgucut cctgtgt                                                      17


<210>  52
<211>  17
<212>  DNA
<213>  Artificial Sequence

<220>
<223>  Antisense oligonucleotide which is a DNA, RNA or DNA/RNA chimeric
       molecule, with at least one nucleotide being optionally modified

<400>  52
gtatgucutc ctgtgta                                                      17


<210>  53

<211>  16
<212>  DNA
<213>  Artificial Sequence

<220>
<223>  Antisense oligonucleotide which is a DNA, RNA or DNA/RNA chimeric
       molecule, with at least one nucleotide being optionally modified

<400>  53
ggtatgucuu ccugtg                                                       16


<210>  54
<211>  16
<212>  DNA
<213>  Artificial Sequence

<220>
<223>  Antisense oligonucleotide which is a DNA, RNA or DNA/RNA chimeric
       molecule, with at least one nucleotide being optionally modified

<400>  54
gtatgucuuc cugtgt                                                       16


<210>  55
<211>  16
<212>  DNA
<213>  Artificial Sequence

<220>
<223>  Antisense oligonucleotide which is a DNA, RNA or DNA/RNA chimeric
       molecule, with at least one nucleotide being optionally modified

<400>  55
tatgtcutcc tgtgta                                                       16


<210>  56
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  56
ggtatgtctu cctgt                                                        15


<210>  57
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  57
gtatgtcttc ctgtg                                                        15

<210>  58
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  58
tatgtctucc tgtgt                                                        15


<210>  59
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  59
atgucutcct gtgta                                                        15


<210>  60
<211>  23
<212>  DNA
<213>  Artificial Sequence

<220>
<223>  primer

<400>  60
gggttttctc aggattgcta tgc                                               23


<210>  61
<211>  22
<212>  DNA
<213>  Artificial Sequence

<220>
<223>  primer

<400>  61
cccactcagt attgacctcc tc                                                22


<210>  62
<211>  18
<212>  DNA
<213>  Artificial Sequence

<220>
<223>  primer

<400>  62
cccttcattg acctcaac                                                     18

<210>  63
<211>  18
<212>  DNA
<213>  Artificial Sequence

<220>
<223>  primer

<400>  63
ttcacaccca tgacgaac                                                    18


<210>  64
<211>  15
<212>  DNA
<213>  Artificial Sequence

<220>
<223>  Antisense oligonucleotide which is a DNA, RNA or DNA/RNA chimeric
       molecule, with at least one nucleotide being optionally modified

<400>  64
cagcttgaac cgggc                                                       15